# EUROPEAN PATENT SPECIFICATION

(11) **EP 4 032 585 B1**
(45) Date of publication and mention of the grant of the patent: **25.02.2026**
(21) Application number: 20864746.1
(22) Date of filing: 08.09.2020
(51) Int. Cl.: A61N 1/40, A61N 1/06, A61N 1/32, A61N 1/08

(54) **SKIN TREATMENT DEVICE USING RADIO FREQUENCY**
VORRICHTUNG ZUR BEHANDLUNG DER HAUT UNTER VERWENDUNG VON RADIOFREQUENZ
DISPOSITIF DE TRAITEMENT DE LA PEAU UTILISANT LA RADIOFRÉQUENCE

(30) Priority: 17.09.2019 KR 20190114144; 16.01.2020 KR 20200006131
(43) Date of publication of application: 27.07.2022
(73) Proprietor: Jeisys Medical Inc., Geumcheon-gu Seoul 08501 (KR)
(72) Inventor: KANG, Dong Hwan, Incheon 22229 (KR)
(74) Representative: dompatent
(86) International application number: PCT/KR2020/012078
(87) International publication number: WO 2021/054664

(56) References cited:
- JP-A- 2018 511 436
- KR-A- 20110 066 324
- KR-A- 20150 049 386
- KR-A- 20190 062 869
- KR-B1- 101 048 506
- US-A1- 2009 018 628
- US-A1- 2009 105 706
- US-B1- 6 413 255
- US-B2- 8 702 691

## Description

### TECHNICAL FIELD

The present invention relates to a skin treatment technology, and more particularly, to a device for treating the skin using a radio frequency (RF) for treatment of the skin or beauty.

### BACKGROUND ART

In general, the skin is the greatest protective organ of the human body that convers the whole of the body and is largely classified into three layers such as an epidermis layer, a dermis layer, and a subcutaneous fat layer.

The epidermis layer that is a layer located on the outermost surface of the skin, includes a stratum corneum, a transparent layer, a granular layer, a polar layer, and a base layer according to the position and function, and is configured to perform the function of protection, defense, secretion, and the like.

The dermis layer is located under the epidermis layer adjacent to the base layer of the epidermis layer and forms the most part of the skin. The dermis layer includes a papillary layer in which moisture, proteins, carbohydrates, mucopolysaccharides, minerals and inorganic salts become jelly-like and capillaries related to blood circulation and lymphatic vessels for carrying lymph are located, and a reticular layer including collagen that is a collagenous fiber related to the wrinkles of the skin, elastin that is an elastic fiber that gives elasticity to the skin, and a matrix.

The subcutaneous fat layer is located between the dermis layer, muscles and bones, includes a large amount of fat and constitutes the lowermost layer of the skin. The subcutaneous fat layer spreads evenly throughout the whole human body and absorbs external pressure and shocks by maintaining elasticity and through a buffering action to prevent damage inside the body and loss of body heat to maintain body temperature.

Supplement injection therapy and electrotherapy are representative treatments that are widely used for skin aging and disease symptoms such as scarring, wrinkles, flexion, melasma, and freckles.

Supplement injection therapy is a treatment, whereby the fibrous tissue under the skin is cut by inserting a needle or a small scalpel under the skin and injects supplements such as autologous fat, platelet-rich plasma (PRP), and fillers to regenerate the skin to its original state.

Electrotherapy is a treatment, whereby disease symptoms are treated by passing an electric current directly to the skin, and functions and uses of electrotherapy vary depending on the waveform of a frequency, the intensity of electric current, an application site, and the like. Recently, many high-frequency electrotherapy methods and low-frequency deep diathermanous devices have been developed and used depending on the applied frequency. In low-frequency electrotherapy, frequencies of 1,000 Hz or less are used, and in high-frequency electrotherapy, frequencies of 10,000 Hz or more are used.

The treatment using high frequency is a method, whereby treatment is performed using deep heat generated in the skin tissue when a high frequency signal is applied to the lesion part of the human body. Deep heat is bioheat generated by rotational, twisting, and collisional motions when the molecules constituting the tissue vibrate and rub against each other whenever the direction of a high-frequency current applied to the human body changes.

In treatment using a high-frequency current, a specific part of the body tissue may be heated without stimulating sensory and motor nerves, and without causing discomfort or muscle contraction in the human body, unlike other current types.

Deep heat generated by a high frequency increases the temperature of the tissue to enhance the functions of cells and increase a blood flow rate, so that various physiological treatment effects such as enhancing metabolism, relieving deep pain, and reducing joint stiffness and beauty care effects such as anti-aging of the skin, scarring, wrinkles, flexion, melasma, freckles, body fat burning, and hair loss treatment and prevention can be shown.

When a high-frequency current flows through a conductor, a phenomenon in which the current flows only near the surface of the conductor, occurs, which is called the skin effect. The skin depth is a numerical representation of the skin effect, and means the penetration depth of the high-frequency current into a material. The skin depth is inversely proportional to each of the conductivity of the material and frequency. In the case of the human skin, the skin depth is about 919 mm when an alternating current (AC) of 1 MHz, which is often used for high-frequency treatment, is applied to the human skin, which means that the high-frequency current excessively penetrates deep into the skin in terms of skin treatment and causes the efficiency of skin treatment to be lowered. It is appropriate that the skin depth is about 0.5 mm for skin treatment. To this end, it may be considered to increase the frequency of the applied high-frequency current, but in order to obtain the skin depth of 0.5 mm, a high-frequency current of about 500 GHz needs to be applied, which is technically very difficult.

US 6 413 255 B1 discloses an apparatus to treat the skin, which includes a template having a tissue interface surface and an energy deliver device coupled to the template. US 2009/018628 A1 discloses an apparatus for treating skin conditions by delivering high frequency energy across large tissue areas. US 8 702 691 B2 discloses a treatment apparatus for delivering energy at multiple selectable depths in tissue.

### SUMMARY OF THE INVENTION

The present invention provides a skin treatment device using a radio frequency.

The present invention also provides a skin treatment device using a radio frequency in which the efficiency of skin treatment is enhanced.

The present invention is defined by the appended independent claim, and preferred aspects of the present invention are defined by the appended dependent claims.

### DETAILED DESCRIPTION

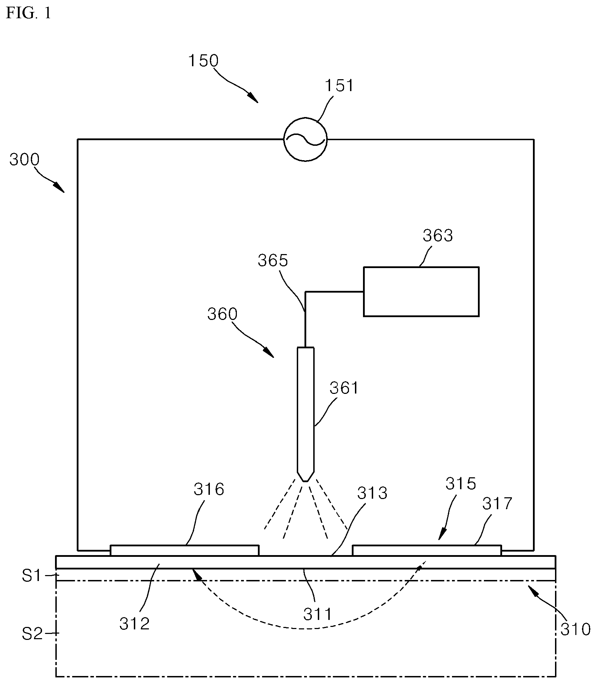

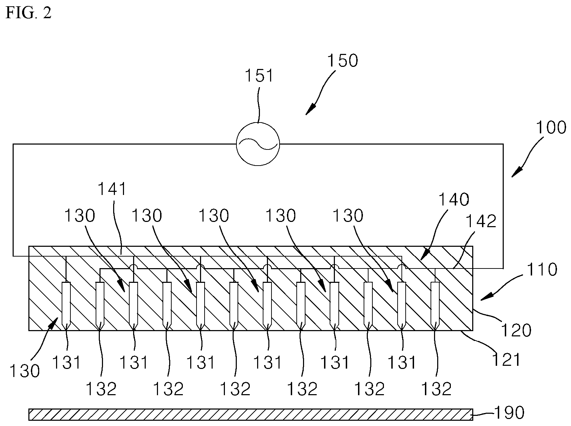

In the following the invention is described with reference to Fig. 2 to 4. A further embodiment, which is not claimed, and shown in Fig. 1 is also disclosed for illustrative purposes. The non-claimed embodiment is as follows: a skin treatment device using a radio frequency, the skin treatment device including a pair of electrodes including: a first electrode and a second electrode and forming a bipolar electrode structure; an RF generation unit which has an output power of 100-500 W, and produces a radio frequency current of 4-15 MHz and supplies same to the first electrode and the second electrode so as to cause a discharge therebetween; a dielectric sheet on which the pair of electrodes is integrally formed and which is disposed to cover a skin portion to be treated, in a contact or non-contact type in the middle of a discharge path between the first electrode and the second electrode; and a cooling part including a nozzle for spraying a cooling fluid so as to cool the dielectric sheet.

According to the present invention, the objectives of the present invention described above can be achieved.

Specifically, a dielectric sheet is arranged between the skin and a pair of electrodes to which a high-frequency current is applied, so that a discharge between the pair of electrodes is performed in a dermis layer of the skin and thus the efficiency of skin treatment is enhanced.

### DESCRIPTION OF THE DRAWINGS

FIG. 1 is a view schematically illustrating the configuration of a skin treatment device using a radio frequency according to a non-claimed embodiment;
FIG. 2 is a view schematically illustrating the configuration of a skin treatment device using a radio frequency according to another embodiment of the present invention;
FIG. 3 is a view schematically illustrating a state in which the skin treatment device using a radio frequency illustrated in FIG. 2 is applied to the skin; and
FIG. 4 is a view schematically illustrating the configuration of an electrode module and an RF generation unit of a skin treatment device using a radio frequency according to another embodiment of the present invention.

### MODE OF THE INVENTION

Hereinafter, the configuration and operation of the embodiments of the present invention will be described in detail with reference to Fig. 2 to 4.

FIG. 1 illustrates the schematic configuration of a skin treatment device using a radio frequency according to a non-claimed embodiment. Referring to FIG**.** 1, a skin treatment device 300 using a radio frequency includes an electrode module 310, an RF generation unit 150 that generates power having a radio frequency and supplies the generated power to the electrode module 310, and a cooling part 360 that supplies a cooling fluid.

The electrode module 310 includes a dielectric sheet 312, and a pair of electrodes 315 integrally formed on the dielectric sheet 312.

The dielectric sheet 312 is formed of a material such as ceramic, and when skin treatment is performed, the dielectric sheet 312 may be in direct contact with the skin to be treated or may be spaced apart from the skin due to an oil or a gel applied to the skin to be treated. That is, the dielectric sheet 312 covers a skin portion to be treated in a contact or non-contact type during skin treatment. A pair of electrodes 315 is integrally formed on the dielectric sheet 312. Although not illustrated, the dielectric sheet 312 is coupled to the pair of electrodes 315 and is supported by using a support body (not illustrated). The dielectric sheet 312 causes a high-frequency discharge generated in the pair of electrodes 315 to be performed mostly in a dermis layer of the skin.

The pair of electrodes 315 is integrally formed on the dielectric sheet 312. In the present embodiment, it will be described that the pair of electrodes 315 is attached to an inner surface 313 of both surfaces of the dielectric sheet 312, which is opposite to an outer surface 311 facing the skin during skin treatment, and is formed integrally. In FIG. 1, one pair of electrodes 315 is illustrated. A plurality of pairs of electrodes 315 may be provided. The pair of electrodes 315 includes a plate-shaped first electrode 316 and a plate-shaped second electrode 317, which are attached to the inner surface 313 of the dielectric sheet 312 and is fixed thereto. An electric potential difference between a first electrode 316 and a second electrode 317 caused by an alternating current (AC) power source is generated by the RF generation unit 150. A bipolar electrode structure is formed by the pair of electrodes 315.

The RF generation unit 150 generates power having a radio frequency and supplies the generated power to the pair of electrodes 315. The RF generation unit 150 includes an AC power source 151 for generating power having a radio frequency. The AC power source 151 generates an electric potential difference between the first electrode 316 and the second electrode 317 of the pair of electrodes 315. Between the first electrode 316 and the second electrode 317, a positive (+) electric potential and a negative (-) electric potential are generated by the RF generation unit 150 while being changed into a radio frequency. Unlike this, one of a first electrode 316 and a second electrode 317 may be configured to serve as a ground electrode, and the polarity of the other one electrode may be configured to be changed. The RF generation unit 150 may generate and supply a pulse current. In the present embodiment, it will be described that the RF generation unit 150 uses 100 to 500 W of radio frequency of 4 to 15 MHz. Because the lower the frequency, the greater the skin penetration depth, when the frequency is lower than 4 MHz, a high-frequency current penetrates to an excessive depth, and it is difficult to implement a frequency higher than 15MHz because it has little effect on the skin in reality.

The cooling part 360 cools the pair of electrodes 315 and the dielectric sheet 312 by spraying and using the cooling fluid to prevent burns of the skin. The cooling part 360 includes a nozzle 361 through which the cooling fluid is sprayed, a cooling fluid storing part 363 in which the cooling fluid is stored, and a connection tube 365 that connects the nozzle 361 to the cooling fluid storing part 363. The nozzle 361 is located at the inner surface 313 of the dielectric sheet 312 and sprays the cooling fluid toward the dielectric sheet 312. The cooling fluid sprayed through the nozzle 361 is stored in the cooling fluid storing part 363. The connection tube 365 connects the nozzle 361 to the cooling fluid storing part 363, and the cooling fluid is supplied to the nozzle 361 through the connection tube 365. Although not illustrated, the cooling part 360 further includes a pump for supplying the cooling fluid stored in the cooling fluid storing part 363 to the nozzle 361, and a valve for controlling spraying of the cooling fluid through the nozzle 361. Although not illustrated, a counter for counting the number of times the cooling fluid is sprayed through the nozzle 361, and a notification unit such as a display for informing the number of times of spraying and/or the number of remaining sprayable times may be further provided. Counting of the number of times of spraying may be performed by detecting the operation of the valve. Because the nozzle 361 sprays the cooling fluid toward the inner surface 313 of the dielectric sheet 312, the sprayed cooling fluid does not come into direct contact with the skin. Thus, a person to be treated may not feel discomfort or the like that may occur when the cooling fluid comes into direct contact with the skin.

Hereinafter, the embodiment shown in FIG. 1 will be described based on an operation with reference to the accompanying drawings.

Referring to FIG. 1, when, in a state in which the dielectric sheet 312 is located to cover a portion of the skin to be treated, the RF generation unit 150 operates to apply an alternating current power having a radio frequency to the first electrode 316 and the second electrode 317 of the pair of electrodes 315, a discharge between the first electrode 316 and the second electrode 317 occurs in a form shown by a dashed-lined arrow. A discharge between the first electrode 316 and the second electrode 317 that form the pair of electrodes 315 is formed on a dermis layer S2 under an epidermis layer S1 of the skin by the dielectric sheet 312 so that the efficiency of skin treatment is enhanced. That is, the skin depth is located in the dermis layer S2 by the dielectric sheet 312 so that a current caused by a discharge does not flow deeper than the dermis layer S2. In the absence of the dielectric sheet 312, the skin depth increases (about 919 mm when 1 MHz is applied), and the current penetrates deeper than the skin depth so that the efficiency of skin treatment is lowered.

FIG. 2 illustrates the schematic configuration of a skin treatment device using a radio frequency according to another embodiment of the present invention. Referring to FIG. 2, a skin treatment device 100 using a radio frequency according to another embodiment of the present invention includes an electrode module 110, an RF generation unit 150 that generates power having a radio frequency and supplies the generated power to the electrode module 110, and a dielectric sheet 190.

The electrode module 110 includes an insulator 120, a plurality of pairs of electrodes 130 installed in the insulator 120, and a wiring structure 140 electrically connected to the plurality of pairs of electrodes 130.

The insulator 120 is an electric insulator, and the plurality of pairs of electrodes 130 are installed in the insulator 120. The insulator 120 may be formed of a flexible material such as Teflon or silicon to correspond to the flexion of the skin. However, the present invention is not limited thereto. The insulator 120 is formed with a generally flat treatment surface 121.

The plurality of pairs of electrodes 130 are installed to be located inside the insulator 120. Each of the plurality of pairs of electrodes 130 includes the first electrode 131 and the second electrode 132. Each of the first electrode 131 and the second electrode 132 has the shape of a rod that extends in a direction generally perpendicular to a treatment surface 121 and has a pointed end. The pointed end of the first electrode 131 and the pointed end of the second electrode 132 are exposed through the treatment surface 121 of the insulator 120. The pointed end of the first electrode 131 and the pointed end of the second electrode 132 preferably may be located on the treatment surface 121 of the insulator 120. An electric potential difference between the first electrode 131 and the second electrode 132 caused by the AC power source is generated by the RF generation unit 150. Because a plurality of pairs of electrodes 130 are provided, a plurality of first electrodes 131 and a plurality of second electrodes 132 are installed in the insulator 120. The plurality of first electrodes 131 may be electrically connected to each other by the wiring structure 140, and the plurality of second electrodes 132 may be electrically connected to each other by the wiring structure 140. The first electrode 131 and the second electrode 132 that form one pair of electrodes 130 are located closer than a first electrode and a second electrode that form another pair of electrodes. In the present embodiment, it has been described that there are six pairs of electrodes 130, but this is just for description of the present invention, and the number of pairs of electrodes 130 may be five or less or seven or more, and this also belongs to the scope of the present invention. In the present embodiment, it has been described that the first electrode 131 and the second electrode 132 have a rod shape, but the present invention is not limited thereto, and any form in which a part of the treatment surface 121 of the insulator 120 is exposed, is possible. In the present embodiment, it has been described that the first electrode 131 and the second electrode 132 are insulated from each other by the insulator 120, but unlike this, the first electrode 131 and the second electrode 132 may be insulated from each other by air without including the insulator 120, and this also belongs to the scope of the present invention.

The wiring structure 140 is electrically connected to the plurality of pairs of electrodes 120. The wiring structure 140 includes a first wiring part 141 for electrically connecting the plurality of first electrodes 131, and a second wiring part 142 for electrically connecting the plurality of second electrodes 132. The first wiring part 141 and the second wiring part 142 are connected to the RF generation unit 150.

The RF generation unit 150 generates power having a radio frequency and the generated power to the plurality of pairs of electrodes 130. The RF generation unit 150 includes an AC power source 151 for producing power having a radio frequency. The AC power source 151 is connected to the first wiring part 141 and the second wiring part 142 so that an electric potential difference between the first electrode 131 and the second electrode 132 of each of the plurality of pairs of electrodes 130 occurs. A positive (+) electric potential and a negative (-) electric potential between the first electrode 131 and the second electrode 132 are generated by the RF generation unit 150 while being changed into a radio frequency. Unlike this, one of the first electrode 131 and the second electrode 132 serves as a ground electrode, and the polarity of the other one electrode may be configured to be changed, and this also belongs to the scope of the present invention. The RF generation unit 150 may generate and supply a pulse current, and this also belongs to the scope of the present invention.

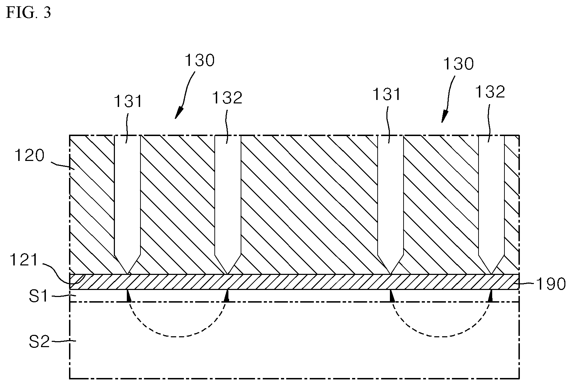

The dielectric sheet 190 is formed of a material such as ceramic, and is located between the treatment surface 121 of the insulator 120 and the epidermis layer S1 of the skin during skin treatment and is used, as illustrated in FIG. 3. In the present embodiment, the dielectric sheet 190 is independently attached to a portion of the skin to be treated to be replaceable for each treatment. However, unlike this, the dielectric sheet 190 is coupled to the electrode module 110 and is used, and this also belongs to the present invention. A high-frequency discharge between the first electrode 131 and the second electrode 132 is performed mostly in the epidermis layer by the dielectric sheet 190. The dielectric sheet 190 may preferably be formed of a flexible material to correspond to the flexion of the skin. The dielectric sheet 190 may come into direct contact with the skin or may be spaced apart from the skin by an oil or a gel applied to the skin. In the present embodiment, it is described that the dielectric sheet 190 is in close contact with the end of the first electrode 131 and the end of the second electrode 132, but the present invention is not limited thereto. The dielectric sheet 190 may be spaced apart from the end of the first electrode 131 and the end of the second electrode 132 at a distance at which radio-frequency can be transferred by an electric field formed by the dielectric sheet 190, and this also belongs to the scope of the present invention.

Hereinafter, the embodiment illustrated in FIG. 2 will be described based on an operation with reference to FIG. 3.

Referring to FIG. 3, the dielectric sheet 190 is located to cover the portion of the skin to be treated, and the insulator 120 is disposed on the dielectric sheet 190. The treatment surface 121 of the insulator 120 comes into close contact with the dielectric sheet 190, and accordingly, ends of the plurality of first electrodes 131 and the plurality of second electrodes 132 exposed through the treatment surface 121 of the insulator 120 come into close contact with the dielectric sheet 190. In this state, when the RF generation unit 150 operates to apply an AC power having a radio frequency to the first electrode 131 and the second electrode 132 of the pair of electrodes 130, a discharge between the end of the first electrode 131 and the end of the second electrode 132 occurs in a form shown by a dashed-lined arrow. The discharge between the first electrode 131 and the second electrode 132 that constitute the pair of electrodes 130 is formed on the dermis layer S2 by the dielectric sheet 190 so that the efficiency of skin treatment is enhanced. That is, the skin depth is located in the dermis layer S2 by the dielectric sheet 190, so that a current caused by the discharge does not flow deeper than the dermis layer S2. In the absence of the dielectric layer 190, the skin depth increases (about 919 mm when 1 MHz is applied), and the current penetrates deeper than the skin depth so that the efficiency of skin treatment is lowered.

When the dielectric sheet 190 is used as an independent member separated from the electrode module (110 of FIG. 2), the dielectric sheet 190 is attached to the portion of the skin to be treated and then, the electrode module 110 is located on the dielectric sheet 190 and is used.

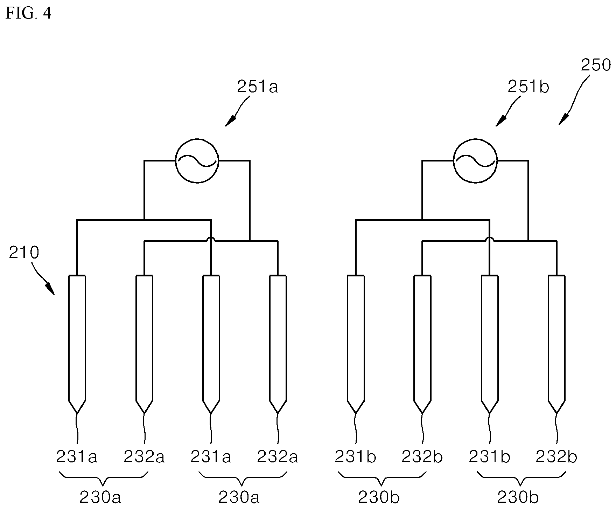

The configuration of an electrode module and an RF generation unit of a skin treatment device using a radio frequency according to another embodiment of the present invention is schematically shown in FIG. 4. Referring to FIG. 4, an electrode module 210 includes a plurality of pairs of first electrodes 230a and a plurality of pairs of second electrodes 230b. Each of the plurality of pairs of first electrodes 230a includes a first A-electrode 231a and a second A-electrode 232a. Each of the plurality of pairs of second electrodes 230b includes a first B-electrode 231b and a second B-electrode 232b. Although not illustrated, the electrode module 210 may further include the insulator 120 shown in FIG. 2, and the plurality of pairs of first electrodes 230a and the plurality of pairs of second electrodes 230b may be installed in the insulator 120. The RF generation unit 250 includes a first AC power source 251a for producing power having a radio frequency applied to the plurality of pairs of first electrodes 230a, and a second AC power source 251b for producing power having a radio frequency applied to the plurality of pairs of second electrodes 230b. In the present embodiment, it is described that there are a plurality of pairs of first electrodes 230a and a plurality of pairs of second electrodes 230b, but unlike this, the number of pairs of first electrodes 230a and the number of pairs of second electrodes 230b may be one, respectively, and this also belongs to the scope of the present invention.

In the embodiment of FIG. 4, it is described that there are two AC power sources. Unlike this, three or more AC power sources are provided to supply power having a radio frequency to a pair of electrodes to which each AC power source corresponds, and this also belongs to the scope of the present invention.

The present invention has been described with reference to exemplary embodiments thereof, but the present invention is not limited thereto. The invention is defined by the appended claims.

## Claims

1. A skin treatment device using a radio frequency (RF), the skin treatment device comprising:
an electrode module (110) comprising an insulator (120), a plurality of pairs of electrodes (130) installed in the insulator (120), and a wiring structure (140) electrically connected to the plurality of pairs of electrodes (130), each pair of the electrodes comprising a first electrode (131) and a second electrode (132);
an RF generation unit (150) which produces power having a radio frequency and supplies same to the electrode module (110) so as to cause a discharge between the first electrode (131) and the second electrode (132) by an electric potential difference generated between the first electrode (131) and the second electrode (132); and
a dielectric sheet (190) configured to be located between the electrode module (110) and an epidermis layer of a skin,
**characterized in that** each of the first electrode (131) and the second electrode (132) has a shape of a rod that extends in a direction generally perpendicular to a treatment surface of the insulator (120) and has a pointed end, and
the pointed end of the first electrode (131) and the pointed end of the second electrode (132) are exposed through the treatment surface of the insulator (120) and come into close contact with the dielectric sheet (190).

2. The skin treatment device of claim 1, wherein the dielectric sheet (190) can be independently attached to a portion of the skin to be treated and be replaceable.

3. The skin treatment device of claim 1, wherein the insulator (120) is formed of a flexible material including Teflon or silicon.

## Patentansprüche

1. Vorrichtung zur Behandlung der Haut unter Verwendung von Radiofrequenz (RF), wobei die Vorrichtung zur Behandlung der Haut aufweist:
ein Elektrodenmodul (110), das einen Isolator (120), eine Vielzahl von Paaren von Elektroden (130), die in dem Isolator (120) installiert sind, und eine Verkabelungsstruktur (140) aufweist, die mit der Vielzahl von Paaren von Elektroden (130) elektronisch verbunden ist, wobei jedes Paar von Elektroden eine erste Elektrode (131) und eine zweite Elektrode (132) aufweist;
eine RF-Erzeugungseinheit (150), die Strom erzeugt, der Radiofrequenz aufweist, und diesen dem Elektrodenmodul (110) zuführt, um eine Entladung zwischen der ersten Elektrode (131) und der zweiten Elektrode (132) durch eine elektrische Potentialdifferenz zu bewirken, die zwischen der ersten Elektrode (131) und der zweiten Elektrode (132) erzeugt wird; und
eine dielektrische Platte (190), die so ausgebildet ist, dass sie sich zwischen dem Elektrodenmodul (110) und einer Epidermisschicht einer Haut befindet,
**dadurch gekennzeichnet, dass** jede der ersten Elektrode (131) und der zweiten Elektrode (132) die Form einer Stange hat, die sich in einer Richtung im Allgemeinen senkrecht zu einer Behandlungsfläche des Isolators (120) erstreckt und ein spitz zulaufendes Ende hat, und
das spitz zulaufende Ende der ersten Elektrode (131) und das spitz zulaufende Ende der zweiten Elektrode (132) durch die Behandlungsfläche des Isolators (120) freigelegt sind und in engen Kontakt mit der dielektrischen Platte (190) kommen.

2. Vorrichtung zur Behandlung der Haut nach Anspruch 1, wobei die dielektrische Platte (190) unabhängig an einem Teil der zu behandelnden Haut angebracht und ausgetauscht werden kann.

3. Vorrichtung zur Behandlung der Haut nach Anspruch 1, wobei der Isolator (120) aus einem flexiblen Material ausgebildet ist, das Teflon oder Silizium enthält.

## Revendications

1. Dispositif de traitement de la peau utilisant une radiofréquence (RF), le dispositif de traitement de la peau comprenant :
un module d'électrodes (110) comprenant un isolateur (120), une pluralité de paires d'électrodes (130) installées dans l'isolateur (120), et une structure de câblage (140) connectée électriquement aux électrodes de la pluralité de paires d'électrodes (130), chaque paire des électrodes comprenant une première électrode (131) et une seconde électrode (132) ;
une unité de génération RF (150) qui produit de la puissance ayant une radiofréquence et qui l'applique au module d'électrodes (110) de façon à provoquer une décharge entre la première électrode (131) et la seconde électrode (132) par une différence de potentiel électrique générée entre la première électrode (131) et la seconde électrode (132) ; et
une feuille diélectrique (190) configurée pour être située entre le module d'électrodes (110) et la couche d'épiderme d'une peau,
**caractérisé en ce que** chacune de la première électrode (131) et de la seconde électrode (132) a une forme de tige qui s'étend dans une direction globalement perpendiculaire à une surface de traitement de l'isolateur (120) et comporte une extrémité pointue, et
l'extrémité pointue de la première électrode (131) et l'extrémité pointue de la seconde électrode (132) sont exposées à travers la surface de traitement de l'isolateur (120) et viennent en contact étroit avec la feuille diélectrique (190).

2. Dispositif de traitement de la peau selon la revendication 1, dans lequel la feuille diélectrique (190) peut être fixée indépendamment à une partie de la peau à traiter et peut être remplacée.

3. Dispositif de traitement de la peau selon la revendication 1, dans lequel l'isolateur (120) est formé d'un matériau flexible comprenant du Téflon ou du silicium.
